# EUROPEAN PATENT SPECIFICATION

(11) **EP 1 713 497 B1**
(45) Date of publication and mention of the grant of the patent: **13.04.2016**
(21) Application number: 05707943.6
(22) Date of filing: 04.02.2005
(51) Int. Cl.: A61K 38/48, A61P 43/00

(54) **USE OF FACTOR VIIA FOR TREATING LATE COMPLICATIONS OF TRAUMA**
VERWENDUNG VON FAKTOR VIIA ZUR BEHANDLUNG DER SPÄTFOLGEN VON TRAUMA
UTILISATION D'UN FACTEUR VIIA DANS LE TRAITEMENT DE COMPLICATIONS TARDIVES LIEES A UN TRAUMATISME

(30) Priority: 05.02.2004 DK 200400178; 01.03.2004 DK 200400339; 01.03.2004 DK 200400341; 16.08.2004 DK 200401240; 16.08.2004 DK 200401239; 08.10.2004 DK 200401552; 08.10.2004 DK 200401553
(43) Date of publication of application: 25.10.2006
(73) Proprietor: Novo Nordisk Health Care AG, Thurgauerstrasse 36/38 8050 Zürich (CH)
(72) Inventor: AXELSEN, Mads, DK-2830 Virum (DK); ERHARDTSEN, Elisabeth, DK-2900 Hellerup (DK); SKOLNICK, Brett, E., Philadelphia, PA 19130 (US)
(74) Representative: Nilsson, Karin Norvin
(86) International application number: PCT/EP2005/050486
(87) International publication number: WO 2005/074974

(56) References cited:
- WO-A-03/007983
- WO-A-03/039581
- WO-A-03/039582
- WO-A-03/039584
- WO-A-03/039590
- SAPSFORD W: "A role for recombinant activated factor VII in trauma?" TRAUMA 2002 UNITED KINGDOM, vol. 4, no. 2, 2002, pages 117-123, XP009046918 ISSN: 1460-4086
- DUTTON RICHARD P ET AL: "Recombinant factor VIIa for control of hemorrhage: early experience in critically ill trauma patients." JOURNAL OF CLINICAL ANESTHESIA. MAY 2003, vol. 15, no. 3, May 2003 (2003-05), pages 184-188, XP002326275 ISSN: 0952-8180
- AITKEN MICHAEL G: "Recombinant factor VIIa." EMERGENCY MEDICINE AUSTRALASIA : EMA. 2004 OCT-DEC, vol. 16, no. 5-6, October 2004 (2004-10), pages 446-455, XP002326503 ISSN: 1742-6731

## Description

### FIELD OF THE INVENTION

The invention discloses prevention of, or minimizing severity of, late complications in trauma patients.

### BACKGROUND OF THE INVENTION

Haemostasis is a complex physiological process which ultimately results in the arrest of bleeding. This is dependent on the proper function of three main components: blood vessels (especially the endothelial lining), coagulation factors, and platelets. Once a haemostatic plug is formed, the timely activation of the fibrinolytic system is equally important to prevent further unnecessary haemostatic activation. Any malfunction of this system (due to a reduced number, or molecular dysfunction, of the haemostatic components or increased activation of the fibrinolytic components) may lead to clinical bleeding such as, e.g., haemorrhagic diathesis of varying severity.

In most physiological situations, haemostasis is triggered by the interaction of circulating activated coagulation factor VII (FVIIa) with tissue factor (TF) subsequent to exposure of TF at the site of an injury. Endogenous FVIIa becomes proteolytically active only after forming a complex with TF. Normally, TF is expressed in the deep layers of the vessel wall and is exposed following injury. This ensures a highly localized activation of coagulation and prevents disseminated coagulation. TF also seems to exist in a non-active form, so-called encrypted TF. The regulation of encrypted versus active TF is still unknown.

TF has in recent years been demonstrated in the circulating blood in a variety of situations such as trauma, sepsis, abdominal surgery. These studies used immunochemically-based methods for the determination of TF (ELISA). Such methods determine both active and inactive TF as well as TF in complex with any other proteins, (such as FVIIa, TFPI, etc.) and thus do not indicate whether the TF found is active or not. The respective subjects were undergoing surgery for idiopathic thoracic scoliosis, an extensive surgical trauma associated with significant tissue injury. Another study failed to demonstrate any TF in the circulation following major orthopaedic surgery (total hip replacement and knee replacement) known to be associated with a high frequency of postoperative deep venous thrombosis (DVT).

Activated recombinant human factor VII (rFVIIa) is indicated for the treatment of bleeding episodes in haemophilia A or B subjects with inhibitors to Factor VIII or Factor IX. When given in high (pharmacological) doses, rFVIIa can bind independently of TF to activated platelets and initiate local thrombin generation which is important for the formation of the initial haemostatic plug.

Uncontrolled bleeding is the major cause of death (39%) in civilian trauma victims. Sixty-five percent (65%) of deaths occur after admission to the hospital and exsanguination is responsible for between 15-40% of hospital deaths in trauma subjects. In subjects with complex liver injuries, the mortality exceeds 40%. There is a correlation between transfusion with blood products and mortality. Many critically ill trauma subjects have profound coagulopathy which correlates to the severity of injury.

The uncontrolled life threatening bleeding and acquired coagulopathy secondary to transfusion, hypothermia and other related causes faced by these subjects may further lead to so-called late complications including pulmonary embolism, Disseminated Intravascular Coagulation (DIC), Acute Myocardial infarction, Cerebral Thrombosis, Multiple organ Failure (MOF), systemic inflammatory response syndrome (SIRS), and Acute Respiratory Distress Syndrome (ARDS), which complications contribute significantly to later deaths of trauma victims.

Sapsford W. *et al.* discloses use of rFVIIa as a procoagulant, its mechanism of action and its potential role in blunt and penetrating trauma.

Dutton Richard P. *et al.* discloses Recombinant Factor VIIa for control of haemorrhage in critically ill trauma patients.

WO2003/039590 discloses composition comprising Factor VII or a Factor VII-related polypeptide and aprotinin or an aprotinin-related polypeptide, and the use thereof for treating bleeding episodes.

WO2003/039582 discloses composition comprising Factor VII or a Factor VII-related polypeptide and epsilon-aminocapronic acid, and the use thereof for treating bleeding episodes.

WO2003/039581 discloses compositions comprising Factor VII or a Factor VII-related polypeptide and tranexamic acid, and the use thereof for treating bleeding episodes.

WO2003/007983 discloses compositions comprising a Factor VII or Factor VII-related polypeptide and a Factor XI or Factor XI-related polypeptide, and the use thereof for treating bleeding episodes.

WO2003/039584 discloses pharmaceutical composition comprising Factor VII or a Factor VII-related polypeptide, and Factor V or a Factor V-related polypeptide, and the use thereof for treating bleeding episodes.

Thus, there is a need in the art for improved methods and compositions for acute treatment of trauma, as well as for prevention and attenuation of late complications that result from trauma itself and from conventional modalities that are used to treat trauma victims.

### SUMMARY OF THE INVENTION

The invention provides the use of Factor VIIa or a Factor VIIa equivalent for the manufacture of a medicament for preventing or attenuating late complications in trauma patients. Typical patients for whom the medicament is used are those suffering from coagulopathic bleedings, including, without limitation, patients who have experienced blunt or penetrating trauma.

In another embodiment, the medicament is used to reduce the severity of late complications. In one embodiment the late complications include one or more of: organ failure, Pulmonary embolism (PE), Acute Respiratory Distress Syndrome (ARDS), Disseminated Intravascular Coagulation (DIC), Acute Myocardial Infarction (AMI), Cerebral Thrombosis (CT), Systemic Inflammatory Response Syndrome (SIRS), infection, Multiple Organ Failure (MOF), Acute Lung Injury (ALI), including death caused by one or more of these syndromes.

The invention also provides the use of Factor VIIa or a Factor VIIa equivalent for the manufacture of a medicament for increasing overall survival of a patient at day 20, preferably day 30 following the start of treatment. In another aspect, the invention provides the use of Factor VIIa or a Factor VIIa equivalent for the manufacture of a medicament for reducing the number of days of hospitalization of a trauma patient, including days in the Intensive Care Unit (ICU), bed confinement, and/or on a ventilator in the period from start of treatment (SOT) to day 20, preferably day 30 following the start of treatment or for reducing the risk of death in a trauma patient. In yet another aspect, the invention provides the use of Factor VIIa or a Factor VIIa equivalent for the manufacture of a medicament for improving lung function in a trauma patient. In one embodiment, (i) an amount of 200 µg/kg of Factor VIIa or Factor VIIa equivalent is administered to the patient at the start of treatment; (ii) about 1 hour later, an amount of about 100 µg/kg of Factor VIIa or Factor VIIa equivalent is administered to the patient; and (iii) about 2 hours later, an amount of about 100 µg/kg of Factor VIIa or Factor VIIa equivalent is administered to the patient.

The invention also provides the use of Factor VIIa, or a Factor VIIa polypeptide that exhibits at least 70% of the specific biological activity of FVIIa, for the manufacture of a medicament for preventing or attenuating multiple organ failure (MOF) or acute respiratory distress syndrome (ARDS) in trauma patients that have received transfusions of less than 10 units of packed red blood cells (PRBC), and wherein said transfusions are made between the time of patient's traumatic injury and the time of administration of Factor VIIa or Factor VIIa equivalent.

### LIST OF FIGURES

**Figure 1** shows the distribution of RBC requirements within the 48-hour observation period following first dose of trial product.
**Figure 2** shows the percentage of patients alive at 48 hours receiving > 12 units of RBC within 48 hours of first dose, which equals > 20 units of RBC inclusive of the 8 pre-dose units
**Figure 3** shows survival curves for blunt and penetrating trauma populations.

### DETAILED DESCRIPTION OF THE INVENTION

The present invention provides uses and compositions that can be used advantageously to prevent or attenuate late complications that trauma patients may experience subsequent to their injury and/or as a result of medical interventions that may be used to treat their injuries. The methods are carried out by administering to a trauma patient Factor VIIa or a Factor VIIa equivalent, in a manner that is effective for preventing or attenuating one or more late complications of trauma. A manner effective for preventing or attenuating late complications may comprise administering a predetermined amount of Factor VIIa or a Factor VIIa equivalent, and/or utilizing a particular dosage regimen, formulation, mode of administration, combination with other treatments, and the like. The efficacy of the methods of the invention in preventing late complications of trauma may be assessed using one or more conventionally used parameters of late complications (see below). Late complications that may be prevented by the methods of the invention, or whose severity may be attenuated, include, without limitation, Pulmonary embolism (PE), Acute Respiratory Distress Syndrome (ARDS), Disseminated Intravascular Coagulation (DIC), Acute Myocardial Infarction (AMI), Cerebral Thrombosis (CT), Systemic Inflammatory Response Syndrome (SIRS), infections, Multiple Organ Failure (MOF), and Acute Lung Injury (ALI), including death caused by one or more of these syndromes.

### Patient selection:

Patients who may benefit by use of the methods of the present invention include, without limitation, patients who have suffered from blunt trauma and/or penetrating trauma. Blunt trauma includes blunt injuries, such as, e.g., those caused by traffic accidents or falls, which could result in one or more of liver injuries, multiple fractures, brain contusions, as well as lacerations of the spleen, lungs, or diaphragm. Blunt trauma is generally accompanied by more extensive tissue damage as compared to penetrating trauma and, consequently, more small vessel bleeding. Penetrating trauma includes penetrating injuries, such as, e.g., those caused by gun shot wounds or stab wounds, which could result in penetration of the inferior vena cava, liver damage, lung injury, injury to prostate, urinary bladder, thorax and liver lacerations, and wounds to the pelvis or chest.

Trauma may cause injury to, and subsequent bleeding from, both larger and smaller vessels. Excessive or massive bleeding in most trauma cases presents a combination of bleeding from vessels needing surgical treatment ("surgical bleeding") and diffuse uncontrolled bleeding from small vessels ("coagulopathic bleeding"). Furthermore, trauma subjects who receive massive transfusions often suffer from coagulopathy and continue to bleed profusely despite intervention with surgical procedures, packing, and embolization of larger vessels.

Bleeding refers to extravasation of blood from any component of the circulatory system and encompasses any bleeding (including, without limitation, excessive, uncontrolled bleeding, i.e., haemorrhaging) in connection with trauma. In one series of embodiments, the excessive bleeding is caused by blunt injury; in another it is caused by penetrating injury. In one series of embodiment, the injury(ies) is/are to the liver, spleen, lungs, diaphragm, head, including the brain. In another series of embodiments, the injury(ies) is/are to the inferior vena cava, liver damage, lung injury, injury to prostate, urinary bladder, thorax and liver lacerations, pelvis or chest, or head, including the brain.

Coagulopathy in trauma is multifactorial, encompassing coagulation abnormalities resembling DIC, caused by systemic activation of coagulation and fibrinolysis; excessive fibrinolysis, which can be evident on the first day in some trauma subjects; and dilutional coagulopathy, which is caused by excessive fluid administration. Some fluids such as hydroxyethyl starch (HES) preparations may directly compromise coagulation. Massive transfusion syndrome results in depletion of coagulation factors and impairment of platelet function. Hypothermia causes a slower enzyme activity of the coagulation cascade and dysfunctional platelets. Metabolic abnormalities, such as acidosis, also compromise coagulation especially when associated with hypothermia.

Non-limiting examples of patients in need of treatment according to the invention include those who exhibit one or more of the following:
- Coagulation abnormalities resembling DIC, caused by systemic activation of coagulation and fibrinolysis
- Excessive fibrinolysis
- Dilutional coagulopathy caused by excessive fluid treatment, including, without limitation, a limited number of platelets and/or an impaired platelet function compared to the platelet count and platelet activity of normal pooled blood
- Receipt of hydroxyethyl starch (HES) preparations
- Hypothermia, a including having body temperature below about 37°C, such as, e.g., below about 36°C, below about 35°C, or below about 34°C
- At least one indication of metabolic abnormalities, including, without limitation, acidosis having a blood pH below about 7.5, such as, e.g., below about 7.4, below about 7.3, below about 7.2, or below about 7.1.

The uses of the present invention can be applied advantageously to any patient who has suffered blunt and/or penetrating trauma that, if left untreated, would result in a significant loss of blood, such as, e.g., over 10% of the patient's total blood volume (loss of over 40% of blood volume is immediately life-threatening.) A normal blood volume represents about 7% of an adult's ideal body weight and about 8-9% of a child's ideal body weight.

In one series of embodiments, patients treated according to the invention are those who have received less than about 10 units of whole blood (WB), packed red blood cells (pRBC), or fresh frozen plasma (FFP) between the time of their traumatic injury and the time of administration of Factor VIIa or Factor VIIa equivalent. A unit of WB typically contains about 450 ml blood and 63 ml of conventional anticoagulant/preservative (having a haematocrit of 36-44%). A unit of pRBC typically contains 200-250 ml of red blood cells, plasma, and conventional anticoagulant/preservative (having a haematocrit of 70-80%). In other embodiments, patients treated according to the invention have received less than about 8 units of WB, pRBC, or FFP, such as, e.g., less than about 5 units, or less than about 2 units, or have not received any blood products prior to administration of Factor VIIa or Factor VIIa equivalent.

In one series of embodiments, patients treated according to the invention do not suffer from a bleeding disorder, whether congenital or acquired, such as, e.g., Haemophilia A, B. or C.

In different embodiments of the invention, patients may be *excluded* from treatment if they have received transfusion of 10 units or more of PRBC, such as, e.g., more than 15, 20, 25, or 30 units, or if they have been diagnosed with a congenital bleeding disorder.

### Factor VIIa and Factor VIIa equivalents:

In practicing the present invention, any Factor VIIa or equivalent may be used that is effective in preventing late complications when administered to a trauma patient. In some embodiments, the Factor VIIa is human Factor VIIa, as disclosed, e.g., in U.S. Patent No. 4,784,950 (wild-type Factor VII). The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa.

Factor VIIa equivalents include, without limitation, Factor VII polypeptides that have either been chemically modified relative to human Factor VIIa and/or contain one or more amino acid sequence alterations relative to human Factor VIIa. Such equivalents may exhibit different properties relative to human Factor VIIa, including stability, phospholipid binding, altered specific activity, and the like.

In one series of embodiments, a Factor VIIa equivalent includes polypeptides that exhibit at least about 10%, preferably at least about 30%, more preferably at least about 50%, and most preferably at least about 70%, of the specific biological activity of human Factor VIIa. For purposes of the invention, Factor VIIa biological activity may be quantified by measuring the ability of a preparation to promote blood clotting using Factor VII-deficient plasma and thromboplastin, as described, e.g., in U.S. Patent No. 5,997,864. In this assay, biological activity is expressed as the reduction in clotting time relative to a control sample and is converted to "Factor VII units" by comparison with a pooled human serum standard containing 1 unit/ml Factor VII activity. Alternatively, Factor VIIa biological activity may be quantified by (i) measuring the ability of Factor VIIa or a Factor VIIa equivalent to produce of Factor Xa in a system comprising TF embedded in a lipid membrane and Factor X. (Persson et al., J. Biol. Chem. 272:19919-19924, 1997); (ii) measuring Factor X hydrolysis in an aqueous system (see, Example 5 below); (iii) measuring the physical binding of Factor VIIa or a Factor VIIa equivalent to TF using an instrument based on surface plasmon resonance (Persson, FEBS Letts. 413:359-363, 1997) and (iv) measuring hydrolysis of a synthetic substrate by Factor VIIa and/or a Factor VIIa equivalent.

Examples of factor VII equivalents include, without limitation, wild-type Factor VII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V158T/E296V/M298Q-FVII, S314E/L305V/V158T/K337A/M298Q-FVII, S314E/L305V/V158T/E296V/K337A-FVII, S314E/L305V/V158D/K337A/M298Q-FVII, S314E/L305V/V158D/E296V/K337A -FVII, S314E/L305V/V158D/E296V/M298Q/K337A-FVII, S314E/L305V/V158T/E296V/M298Q/K337A-FVII, K316H/L305V/K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305V/V158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A -FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A-FVII, K316Q/L305V/V158D/E296V/M298Q/K337A-FVII, and K316Q/L305V/V158T/E296V/M298Q/K337A-FVII.

In some embodiments, the factor VII equivalent is V158D/E296V/M298Q-FVII.

### Preparations and formulations:

The present invention encompasses therapeutic administration of Factor VIIa or Factor VIIa equivalents, which is achieved using formulations that comprise Factor VIIa preparations. As used herein, a "Factor VII preparation" refers to a plurality of Factor VIIa polypeptides or Factor VIIa equivalent polypeptides, including variants and chemically modified forms, that have been separated from the cell in which they were synthesized, whether a cell of origin or a recombinant cell that has been programmed to synthesize Factor VIIa or a Factor VIIa equivalent.

Separation of polypeptides from their cell of origin may be achieved by any method known in the art, including, without limitation, removal of cell culture medium containing the desired product from an adherent cell culture; centrifugation or filtration to remove non-adherent cells; and the like.

Optionally, Factor VII polypeptides may be further purified. Purification may be achieved using any method known in the art, including, without limitation, affinity chromatography, such as, e.g., on an anti-Factor VII antibody column (see, e.g., Wakabayashi et al., J. Biol. Chem. 261:11097, 1986; and Thim et al., Biochem. 27:7785, 1988); hydrophobic interaction chromatography; ion-exchange chromatography; size exclusion chromatography; electrophoretic procedures (*e.g*., preparative isoelectric focusing (IEF), differential solubility (*e.g.,* ammonium sulphate precipitation), or extraction and the like. See, generally, Scopes, Protein Purification, Springer-Verlag, New York, 1982; and Protein Purification, J.-C. Janson and Lars Ryden, editors, VCH Publishers, New York, 1989. Following purification, the preparation preferably contains less than about 10% by weight, more preferably less than about 5% and most preferably less than about 1%, of non-Factor VII proteins derived from the host cell.

Factor VII and Factor VII-related polypeptides may be activated by proteolytic cleavage, using Factor XIIa or other proteases having trypsin-like specificity, such as, e.g., Factor IXa, kallikrein, Factor Xa, and thrombin. See, e.g., Osterud et al., Biochem. 11:2853 (1972); Thomas, U.S. Patent No. 4,456,591; and Hedner et al., J. Clin. Invest. 71:1836 (1983). Alternatively, Factor VII may be activated by passing it through an ion-exchange chromatography column, such as Mono Q® (Pharmacia) or the like. The resulting activated Factor VII may then be formulated and administered as described below.

Pharmaceutical compositions or formulations for use in the present invention comprise a Factor VIIa preparation in combination with, preferably dissolved in, a pharmaceutically acceptable carrier, preferably an aqueous carrier or diluent. A variety of aqueous carriers may be used, such as water, buffered water, 0.4% saline, 0.3% glycine and the like. The preparations of the invention can also be formulated into liposome preparations for delivery or targeting to the sites of injury. Liposome preparations are generally described in, e.g., U.S. Patents Nos. 4,837,028, 4,501,728, and 4,975,282. The compositions may be sterilised by conventional, well-known sterilisation techniques. The resulting aqueous solutions may be packaged for use or filtered under aseptic conditions and lyophilised, the lyophilised preparation being combined with a sterile aqueous solution prior to administration.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

### Treatment regimen:

In practicing the present invention, Factor VIIa or the Factor VIIa equivalent may be administered to a patient as a single dose comprising a single-dose-effective amount for preventing or treating late complications, or in a staged series of doses which together comprise an effective amount for preventing or treating late complications. An effective amount of Factor VIIa or the Factor VIIa equivalent (see below) refers to the amount of Factor VIIa or equivalent which, when administered in a single dose or in the aggregate of multiple doses, or as part of any other type of defined treatment regimen, produces a measurable statistical improvement in outcome, as evidenced by at least one clinical parameter associated with the late complications of trauma (see below). When Factor VIIa equivalents are administered, an effective amount may be determined by comparing the coagulant activity of the Factor VIIa equivalent with that of Factor VIIa and adjusting the amount to be administered proportionately to the predetermined effective dose of Factor VIIa.

Administration of Factor VIIa or a Factor VIIa equivalent according to the present invention is preferably initiated within about 6 hours after occurrence of the traumatic injury, such as, e.g., within about 4 hours, within about 2 hours, or within about 1 hour.

Administration of a single dose refers to administration of an entire dose of Factor VIIa or the Factor VIIa equivalent as a bolus over a period of less than about 5 minutes. In some embodiments, the administration occurs over a period of less than about 2.5 minutes, and, in some, over less than about 1 min. Typically, a single-dose effective amount comprises at least about 40 ug/kg human Factor VIIa or a corresponding amount of a Factor VIIa equivalent, such as, at least about 50 ug/kg, 75 ug/kg, or 90 ug/kg, or at least 150 ug/kg Factor VIIa.

In some embodiments, following administration of a single dose of Factor VIIa or a Factor VIIa equivalent according to the invention, the patient receives no further Factor VIIa or Factor VIIa equivalent for an interval of at least about 15 minutes. In some embodiments the post-administration interval is at least about 30 minutes, such as at least about 45 minutes, at least about 1 hour, at least about 1.5 hours, or at least about 2 hours.

In other embodiments, the patient receives Factor VIIa or Factor VIIa equivalent according to the following regimen: (i) The patient receives a first amount of Factor VIIa or Factor VIIa equivalent comprising at least about 40 ug/kg; (ii) after a period of at least about 30 minutes, a second amount of Factor VIIa or Factor VIIa equivalent is administered, the amount comprising at least about 40 ug/kg; and (iii) after a period of at least about 30 minutes from administration of the second dose, a third amount of Factor VIIa or Factor VIIa equivalent is administered, the amount comprising at least about 40 ug/kg. After a period of at least about 30 minutes following the administration of the third amount, the patient may then receive a further (fourth) amount of Factor VIIa or Factor VIIa equivalent comprising at least about 40 ug/kg.

In other embodiments, the first amount of Factor VIIa or Factor VIIa equivalent comprises at least about 100 ug/kg, such as at least about 150 ug/kg or at least about 200 ug/kg; in other embodiments, the second amount of Factor VIIa or Factor VIIa equivalent comprises at least about 75 ug/kg, such as at least about 90 ug/kg; in other embodiments, the third (and optionally fourth) amount of Factor VIIa or Factor VIIa equivalent comprises at least about 75 ug/kg, such as at least about 90 ug/kg.

In one embodiment, the first dose comprises about 200 ug/kg, the second dose about 100 ug/kg, and the third (and optionally fourth) dose about 100 ug/kg.

In other embodiments, the patient receives the second amount of Factor VIIa or Factor VIIa equivalent after a period of at least about 45 minutes from the first administration, such as at least about 1 hour, at least about 1.5 hours, at least about 2 hours, at least about 2.5 hours, or at least about 3 hours.

In other embodiments, the patient receives the third (and optionally fourth) amount of Factor VIIa or Factor VIIa equivalent after a period of at least about 45 minutes from the previous administration, such as at least about 1 hour, at least about 1.5 hours, at least about 2 hours, at least about 2.5 hours, or at least about 3 hours.

In one embodiment, the patient receives a first dose comprising about 200 ug/kg; after a period of about 1 hour, the patient receives a second dose comprising about 100 ug/kg, and after a period of about 3 hours from the first dose, the patient receives a third dose comprising about 100 ug/kg.

The following table illustrates different non-limiting embodiments of the invention:

| Time post-injury | 1^{st} Dose | Time to 2^{nd} dose | 2^{nd} Dose (optional) | Time to 3^{rd} dose | 3^{rd} Dose (optional) | Additional Doses |
|---|---|---|---|---|---|---|
| 0 - ≤1h | >40 mcg/kg | 0-1h | >40mcg/kg | 0-1h | >40mcg/kg | As needed |
| | | 1-2h | | 1-2h | | |
| | | ≥3 h | | ≥3 h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |
| > 1 - ≤3h | >40 mcg/kg | 0-1h | >40mcg/kg | 0-1h | >40mcg/kg | As needed |
| | | 1-2h | | 1-2h | | |
| | | ≥3 h | | ≥3 h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |
| > 3 - > 6h | >50mcg/kg | 0-1h | >50mcg/kg | 0-1h | >50mcg/kg | As needed |
| | | 1-2h | | 1-2h | | |
| | | ≥3 h | | ≥3 h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |
| >6 - < 12h | >50mcg/kg | 0-1h | >50mcg/kg | 0-1h | >50mcg/kg | As needed |
| | | 1-2h | | 1-2h | | |
| | | ≥3 h | | ≥3 h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcg/kg | 0-1h | 50-100 mcg/kg | 0-1h | 50-100 mcg/kg | |
| | | 1-2h | | 1-2h | | |
| | | ≥3 h | | ≥3 h | | |
| >12 h | >50mcg/kg | 0-1h | >50mcg/kg | 0-1h | >50mcg/kg | As needed |
| | | 1-2h | | 1-2h | | |
| | | ≥3 h | | ≥3 h | | |
| | 100 mcg/kg | 0-1h | 50-100 mcg/kg | 0-1h | 50-100 mcg/kg | |
| | | 1-2h | | 1-2h | | |
| | | ≥3 h | | ≥3 h | | |
| | ≥150 mcq/kq | 0-1h | 50-100 mcg/kg | 0-1h | 50-100 mcg/kg | |
| | | 1-2h | | 1-2h | | |
| | | ≥3 h | | ≥3 h | | |

It will be understood that the effective amount of Factor VIIa or Factor VIIa equivalent, as well as the overall dosage regimen, may vary according to the patient's haemostatic status, which, in turn, may be reflected in one or more clinical parameters, including, e.g., relative levels of circulating coagulation factors; amount of blood lost; rate of bleeding; haematocrit, and the like. It will be further understood that the effective amount may be determined by those of ordinary skill in the art by routine experimentation, by constructing a matrix of values and testing different points in the matrix.

For example, in one series of embodiments, the invention encompasses (i) administering a first dose of Factor VIIa or a Factor VIIa equivalent; (ii) assessing the patient's coagulation status after a predetermined time; and (iii) based on the assessment, administering a further dose of Factor VIIa or Factor VIIa equivalent if necessary. Steps (ii) and (iii) may be repeated until satisfactory haemostasis is achieved.

According to the invention, Factor VIIa or a Factor VIIa equivalent may be administered by any effective route, including, without limitation, intravenous, intramuscular, subcutaneous, mucosal, and pulmonary routes of administration. Preferably, administration is by an intravenous route.

### Combination treatments:

The present invention encompasses combined administration of an additional agent in concert with Factor VIIa or a Factor VIIa equivalent. In some embodiments, the additional agent comprises a coagulant, including, without limitation, a coagulation factor such as, e.g., Factor VIII, Factor IX, Factor V, Factor XI, or Factor XIII; or an inhibitor of the fibrinolytic system, such as, e.g., PAI-1, aprotinin, ε -aminocaproic acid or tranexamic acid.

It will be understood that, in embodiments comprising administration of combinations of Factor VIIa with other agents, the dosage of Factor VIIa or Factor VIIa equivalent may on its own comprise an effective amount and additional agent(s) may further augment the therapeutic benefit to the patient. Alternatively, the combination of Factor VIIa or equivalent and the second agent may together comprise an effective amount for preventing late complications associated with trauma. It will also be understood that effective amounts may be defined in the context of particular treatment regimens, including, e.g., timing and number of administrations, modes of administrations, formulations, etc.

### Treatment outcomes:

The present invention provides uses and compositions for preventing and/or attenuating one or more late complications of trauma. Late complications include, without limitation, Pulmonary embolism (PE), Acute Respiratory Distress Syndrome (ARDS), Disseminated Intravascular Coagulation (DIC), Acute Myocardial Infarction (AMI), Cerebral Thrombosis (CT), Systemic Inflammatory Response Syndrome (SIRS), infections, Multiple Organ Failure (MOF), and Acute Lung Injury (ALI), including death caused by one or more of these syndromes.

In practicing the present invention, late complications may be assessed using conventional methods, such as, e.g., the Scores described in Tables 1 to 5 herein. Assessments may be performed at least about 20 days from the start of treatment according to the invention, such as, e.g., at least about 30 days, at least about 35 days, or at least about 40 days from the start of treatment.

Organ damage or organ failure encompass, without limitation, damage to the structure and/or damage to the functioning of the organ in kidney, lung, adrenal, liver, bowel, cardiovascular system, and/or haemostatic system. Examples of organ damage include, but are not limited to, morphological/structural damage and/or damage to the functioning of the organ such as, for example accumulation of proteins (for example surfactant) or fluids due to pulmonary clearance impairment or damage to the pulmonary change mechanisms or alveolo-capillary membrane damage. The terms "organ injury", "organ damage" and "organ failure" may be used interchangeably. Normally, organ damage results in organ failure. By organ failure is meant a decrease in organ function compared to the mean, normal functioning of a corresponding organ in a normal, healthy person. The organ failure may be a minor decrease in function (e.g., 80-90% of normal) or it may be a major decrease in function (e.g., 10-20% of normal); the decrease may also be a complete failure of organ function. Organ failure includes, without limitation, decreased biological functioning (e.g., urine output), e.g., due to tissue necrosis, loss of glomeruli (kidney), fibrin deposition, haemorrhage, oedema, or inflammation. Organ damage includes, without limitation, tissue necrosis, loss of glomeruli (kidney), fibrin deposition, haemorrhage, oedema, or inflammation.

Lung damage encompasses, but is not limited to, morphological/structural damage and/or damage to the functioning of the lung such as, for example accumulation of proteins (for example surfactant) or fluids due to pulmonary clearance impairment or damage to the pulmonary change mechanisms or alveolo-capillary membrane damage. The terms "lung injury", "lung damage" and "lung failure" may be used interchangeably.

Methods for testing organ function and efficiency, and suitable biochemical or clinical parameters for such testing, are well known to the skilled clinician.

Such markers, or biochemical parameters of organ function are, for example:

| | |
|---|---|
| Respiration: | PaO2/FiO2 ratio |
| Coagulation: | Platelets |
| Liver: | Bilirubin |
| Cardiovascular: | Blood pressure and need for vasopressor treatment |
| Renal: | Creatinine and urine output |

Other clinical assessments comprise ventilator free days, organ failure free days, vasopressor treatment free days, SOFA score and Lung Injury Score evaluation as well as vital signs.

Methods for testing for coagulophathy or inflammation are also well known to the skilled clinician. Such markers of coagulatory or inflammatory state are, for example, PTT, Fibrinogen depletion, elevation in TAT complexes, ATIII activity, IL-6, IL-8, or TNFR-1.

Chronic organ damage encompasses, but is not limited to, the long-term damage that may result from ARDS. This residual impairment, in particular of pulmonary mechanics, may include, without restriction, mild restriction, obstruction, impairment of the diffusing capacity for carbon monoxide, or gas-exchange abnormalities with exercise, fibrosing alveolitis with persistent hypoxemia, increased alveolar dead space, and a further decrease in alveolar or pulmonary compliance. Pulmonary hypertension, owing to obliteration of the pulmonary-capillary bed, may be severe and lead to right ventricular failure.

In the present context, prevention includes, without limitation, the attenuation, elimination, minimization, alleviation or amelioration of one or more symptoms or conditions associated with late complications associated with trauma, including, but not limited to, the prevention of further damage to and/or failure of organs already subject to some degree of organ failure and/or damage, as well as the prevention of damage and/or failure of further organs not yet subject to organ failure and/or damage. Examples of such symptoms or conditions include, but are not limited to, morphological/structural damage and/or damage to the functioning of organs such as, but not limited to, lung, kidney, adrenal, liver, bowel, cardiovascular system, and/or haemostatic system. Examples of such symptoms or conditions include, but are not limited to, morphological/structural damage and/or damage to the functioning of the organs such as, for example, accumulation of proteins (for example surfactant) or fluids due to pulmonary clearance impairment or damage to the pulmonary exchange mechanisms or damage to the alveolo-capillary membrane, decreased urine output (kidney), tissue necrosis, loss of glomeruli (kidney), fibrin deposition, haemorrhage, oedema, or inflammation.

Attenuation of organ failure or damage encompasses any improvement in organ function as measured by at least one of the well known markers of function of said organs (see Tables 1 to 4) compared to the corresponding value(s) found in trauma patients not being treated in accordance with the present invention.

Prevention also includes preventing the development of Acute Lung Injury (ALI) into ARDS. ALI is defined by the following criteria (Bernard et al., Am.J.Respir.Crit.Care Med 149: 818-24, 1994): acute onset; bilateral infiltrates on chest radiography; pulmonary-artery wedge pressure of ≤ 18 mm Hg or the absence of clinical evidence of left atrial hypertension; and PaO₂: FiO₂ of ≤ 300. ARDS is defined by the following criteria (Bernard et al., Am.J.Respir.Crit.Care Med 149: 818-24, 1994): acute onset; bilateral infiltrates on chest radiography; pulmonary-artery wedge pressure of ≤ 18 mm Hg or the absence of clinical evidence of left atrial hypertension, and PaO₂:FiO₂ of ≤ 200. (PaO₂ denotes partial pressure of arterial oxygen, and FiO₂ fraction of inspired oxygen).

### Measurement of Late Complications:

Following are non-limiting examples of methods for assessing the incidence and severity of late complications of trauma.
1. The Glasgow Coma Score is determined as follows

| **Glasgow Coma Scale** | | |
|---|---|---|
| **Eye Opening (E)** | **Verbal Response (V)** | **Motor Response (M)** |
| 4=Spontaneous | 5=Normal conversation | 6=Normal |
| 3=To voice | 4=Disoriented conversation | 5=Localizes to pain |
| 2=To pain | 3=Words, but not coherent | 4=Withdraws to pain |
| 1=None | 2=No words...only sounds | 3=Decorticate posture |
| | 1=None | 2=Decerebrate |
| | | 1=None |
| | | **Total = E+V+M** |

| | | |
|---|---|---|
| Normal = 15 Vegetative = 0 | | |

2. The Multiple Organ Failure (MOF) score is determined as follows:

**Multiple Organ Failure Score**

| Multiple Organ Failure | | An MOF score of 4 or more | | |
|---|---|---|---|---|
| | Grade 0 | Grade 1 Dysfunction | Grade 2 Dysfunction | Grade 3 Dysfunction |
| Pulmonary^{a} | Normal | ARDS score >5 | ADRS score >9 | ARDS score >13 |
| Renal | Normal | Creatinine > 1.8 mg/dL | Creatinine > 2.5 mg/dL | Creatinine > 5 mg/dL |
| Hepatic^{b} | Normal | Bilirubin > 2 mq/dL | Bilirubin > 4 mq/dL | Bilirubin > 8 mq/dL |
| Cardiac^{c} | Normal | Minimal inotropes | Moderate inotropes | High inotropes |
| ^{a}ARDS score=A+B+C+D+E, PCWP ≤ 18 cm H₂0, or clinical setting where high PCWP is not anticipated. | | | | |
| ^{b}Biliary obstruction and resolving haematoma excluded. | | | | |
| ^{c}Cardiac index < 3.0 L/min/m2 requiring inotropic support. Minimal dose, dopamine or dobutamine <5 µg/kg/min; moderate dose, dopamine or dobutamine 5-15 µg/kg/min; high dose, greater than moderate doses of above agents. | | | | |

| | | | | |
|---|---|---|---|---|
| Healthy =0 Severe = 15 | | | | |

3. The ARDS Score is determined as follows:

| ARDS Score | |
|---|---|
| A. Pulmonary findings by plain chest radiography | D. Positive and expiratory pressure (cm H₂0) |
| | 0=<6 |
| 0=Normal | 1=6-9 |
| 1=Diffuse, mild interstitial marking/opacities | 2=30-39 |
| | 3=14-17 |
| 3=Diffuse, moderate airspace consolidation | 4=>17 |
| 4=Diffuse, severe airspace consolidation | |
| B. Hypoxemia - Pao₂/Fio₂ (mmHg) 0=Normal | E. Static compliance (mL/cmH₂0) |
| | 0=>50 |
| 1=175-250 | 1=40-50 |
| 2=125-174 | 2=30-39 |
| 3=80-124 | 3=20-29 |
| 4=<80 | 4=<20 |
| C. Minute ventilation (L/min) | |
| 0=<11 | |
| 1=<11-13 | |
| 2=<14-16 | |
| 3=<17-20 | |
| 4=>20 | |

| | |
|---|---|
| Normal =0 Severe = 20 | |

4. The SIRS Score is determined as follows:

**Systemic Inflammatory Response Syndrome Score**

| A SIRS score (1 to 4) calculated for each subject. |
|---|
| One point for each component present: |
| • fever or hypothermia |
| • tachypnea |
| • tachycardia |
| • leukocytosis |
| SIRS is present when two or more of the following criteria are met: |
| • temperature greater than 38°C or less than 36°C |
| • heart rate greater than 90 beats per minute |
| • respiratory rate greater than 20 breaths per minute or PaCO₂ less than 32 |
| • white blood cell count greater than 12,000/mm³ or less than 4,000/,mm³ or presence of 10% bands |

| |
|---|
| Normal = 0 Severe =4 |

5. DIC is measured as follows:

**DIC**

| Term: | Definition: |
|---|---|
| Disseminated Intravascular Coagulation | Clinical history of: an intense clotting stimulus and shock (infection, trauma, tissue damage, surgery) followed by bleeding. Blood tests: fibrinogen ≤ 150 mg/dL |
| | platelet count < 150,000//mm³ or drop of: 100,000/mm³ from last valve D-dimer > 500 µg/L |

In one series of embodiments, the practice of the present invention results in one or more of the following clinical outcomes:
- A Glasgow Coma Score of greater than about 9 when measured 20 days after start of treatment;
- A Glasgow Coma Score of greater than about 11 when measured 30 days after start of treatment;
- A Glasgow Coma Score of greater than about 13 when measured 40 days after start of treatment;
- An MOF Score of less than about 4 when measured 20 days after start of treatment;
- An MOF Score of less than about 3 when measured 30 days after start of treatment;
- An MOF Score of less than about 2 when measured 40 days after start of treatment;
- An ARDS Score of less than about 8 when measured 20 days after start of treatment;
- An ARDS Score of less than about 6 when measured 30 days after start of treatment;
- An ARDS Score of less than about 4 when measured 40 days after start of treatment;
- An SIRS Score of less than about 3 when measured 20 days after start of treatment;
- An SIRS Score of less than about 2 when measured 30 days after start of treatment;
- An SIRS Score of less than about 1 when measured 40 days after start of treatment:
- Any combination of any of the above Glasgow Coma Scores, MOF Scores, ARDS Scores, and/or SIR Scores.

### Other indices of treatment:

The efficacy of the methods of the present invention may also be assessed using other clinical parameters, including, without limitation, reduction in any one or more of the following parameters relative to a similar patient who has not been administered Factor VIIa or a Factor VIIa equivalent according to the invention: a reduction in units of blood, plasma, red blood cells, packed red blood cells, or volume replacement products that need to be administered; a decrease in the number of days of hospitalization after suffering a trauma, including a decrease in the number of days that a patient may spend in an intensive care unit (ICU) and a decrease in the number of days in which certain interventions (such as, e.g., a ventilator) are required. Non-limiting examples of outcomes include: (i) a reduction in the units of blood, plasma, red blood cells, packed red blood cells, or volume replacement products that need to be administered by at least about 2 units, 4 units, or 6 units; (ii) a decrease in ICU days by 1 day, 2 days, or 4 days; (iii) a reduction on the number of days on a ventilator by 1 day, 2 days, or 4 days; (iv) a reduction in the total days of hospitalization by 2 days, 4 days, or 8 days.

The following examples are intended as non-limiting illustrations of the present invention.

### Example 1: Factor VIIa administration to trauma victims

The following study was performed in order to assess efficacy and safety of recombinant activated coagulation factor VII (rFVIIa, NovoSeven^{®}) as adjunctive therapy for bleeding control in severe trauma

### Methods

A multicenter, randomized, double-blind trial compared rFVIIa with placebo. Study product was administered as 3 i.v. injections (200, 100 and 100 µg/kg) at time 0, 1 and 3h after transfusion of 8 units of red blood cells (RBC). Patients were monitored for 48 hours after dosing with 30-day follow-up. Standard local hospital treatment was given throughout. Blunt and penetrating groups were separately analysed.

### Results

In total, 143 blunt and 134 penetrating patients were analyzed. In patients with blunt trauma (Injury Severity Score mean ± SD: 33±13), there was a trend to decreased RBC transfusion within 48h of dosing (primary endpoint) in the rFVIIa group vs placebo when adjusting for patients who died within 48h (p=0.07). Excluding deceased patients, the reduction in RBC was significant (p=0.02). In particular, fewer patients in the rFVIIa group received massive transfusion (>20 RBC units). Fewer patients with predefined critical complications were observed with rFVIIa in blunt trauma (Table). For patients with penetrating trauma, transfusion results were similar but not statistically significant. The number of thromboembolic events was similar between treatment groups.

### Conclusions

rFVIIa showed a good safety profile in this high-risk trauma population. RBC requirements were significantly reduced with rFVIIa in blunt trauma. Trends to reduced complications warrant further investigation.

**Table: Patients with critical events within 30 days (blunt group)**

| | **Placebo (N=74)** | **rFVIIa (N=69)** |
|---|---|---|
| Multiple Organ Failure | 7 (9%) | 3 (4%) |
| Acute Respiratory Distress Syndrome | 12 (16%) | 3 (4%) |
| Death | 22 (30%) | 17 (25%) |
| ICU-free time | Mean 10.5 d | Mean 12.6 d |
| Ventilator-free time | Mean 13.7 d | Mean 15.4 d |

Results from blunt trauma indicate that patients treated with NovoSeven® have fewer complications and spend less time in intensive care units than patients receiving conventional treatment and also that overall mortality was lower in the group treated with NovoSeven®.

### Example 2: Efficacy of Factor VIIa given in conjunction with standard therapy in the treatment of trauma

### TRIAL DESIGN:

A multi-centre, randomised, double-blind, parallel group, placebo-controlled trial was conducted in subjects with severe blunt and/or penetrating trauma injuries. Subjects were recruited for screening upon admittance to the trauma centre. In conjunction with the trial product, they received standard treatments for injuries and bleeding and any other procedures deemed necessary by the physician in charge of coordinating the trauma team. The trial is comprised of two treatments arms. Eligible subjects, upon receiving 6 units of PRBC within a 4-hour period, will be equally allocated to one of the following arms:
- Standard therapy in conjunction with three single doses (volume equal to 200 µg/kg + 100 µg/kg + 100 µg/kg) of placebo administered over a 3 hour period
- Standard therapy in conjunction with three single doses (200 µg/kg + 100 µg/kg + 100 µg/kg) of rFVIIa administered over a 3 hour period

The first dose of rFVIIa or placebo (trial product) were administered once the subject had received 8 units of PRBC and followed 1 hour later by the second dose and an additional 2 hours later by the third and final dose of trial product. The trial drug were given to subjects who in the opinion of the attending surgeon required more transfusion than 8 units of PRBC. A 48-hour observation period, starting upon administration of the first dose, as well as a 30-day follow-up assessment, were conducted. The trial product was administered intravenously as a slow bolus injection. Specific procedures such as physical examination, laboratory assessment and adverse event evaluation were conducted throughout the trial. The subjects were monitored throughout the study for several endpoints including number of PRBC units required, adverse events, survival, and changes in coagulation related parameters.

In order to evaluate the mortality due to haemorrhage, a sequential analysis of every set of 20 subjects treated was performed starting when mortality data from first 100 subjects were available. Safety was monitored and evaluated continuously taking into account all SAEs as they were reported during the trial.

### TRIAL PRODUCTS:

Activated recombinant human FVII (rFVIIa) and placebo will be supplied as freeze-dried powder in single use vial of 2.4 mg to be reconstituted with sterile water for Ph.Eur. injection.

### TRIAL POPULATION:

Approximately 280 subjects (140 per treatment arm), 16 years or older, with severe blunt and/or penetrating trauma injuries were enrolled.

### Inclusion Criteria

Subjects entering the trial met the following inclusion criteria:
1. Injury(ies) due to a blunt and or penetrating trauma.
2. Receipt of 6 units of PRBC within a 4 hour period following admittance to the trauma centre
3. Receipt of 8 units of PRBC upon administration of trial drug.
4. Known age of ≥16 or legally of age according to local law and ≤65.

### Exclusion Criteria:

Subjects meeting the following criteria were excluded from the study:
   1. Prehospital cardiac arrest.
   2. Cardiac arrest in the ER or OR.
   3. Gunshot wound to the head.
   4. Glasgow Coma Scale < 8.
   5. Base deficit of > 15 mEq/l or severe acidosis (pH<7.0.).
   6. Transfusion of 8 units or more of ^{PRBC} prior to arrival in trauma centre.
   7. Known congenital bleeding disorder.
   8. Currently participating or has participated in another investigational drug trial within the last 30 days.
   9. Known pregnancy or positive pregnancy test at enrolment.
   10. Previous participation in this trial.
   11. Known treatment with vitamin K antagonist, low-dose heparin before trial drug is given.
   12. Injury sustained ≥12 hours prior to randomisation.
   13. Estimated weight >130kg.

### ASSESSMENTS:

**Treatment efficacy is based** on the evaluation of the following variables for the period from SOT to 48 hours:
- Timing and number of deaths due to bleeding and all other causes.
- Timing and number of transfusion units of the following blood products administered: PRBC (timing)

### FFP

### Platelets

### Cryoprecipitate

- Number of times subject is taken to surgery due to bleeding.
- Time interval between first dose of study drug and reaching normal range of coagulation PT, normal temperature, and acid base status.
- Pharmacokinetic evaluations and population pharmacokinetic evaluation
- Overall survival at Day 30
- Timing and number of complications including MOF, ARDS, and infections occurring from SOT to Day 30.
- Number of days of hospitalisation including days in the Intensive Care Unit (ICU), bed confinement and/or on a ventilator in the period from SOT to Day 30.

### Prior to Onset of Treatment (Treatment Period 0)

### Blood sampling was performed for:

FVII:C (cf. below)
Coagulation related parameters and haematology (cf. below)
PT (cf. below)
Blood chemistry (cf.below)

### After first trial product administration and the following 24 hours.

### The following was recorded and/or investigated:

Mortality and time of death
Vital signs at 30 min, 1, 2, 4, 6, 8, 12, 18 and 24 hours (cf. below). Glasgow Coma Score only at 24 hours.
Number of transfusion product units required. (cf. below).
I.V. fluid including the composition, e.g., colloid, crystalloids (cf. below).
Number of times subject is taken to surgery and reason for surgery (cf.below). Adverse events).
ARDS, infection, MOF.

Blood sampling was performed for:
Coagulation related parameters at 1, 4, 8, 12, and 24 hours (cf below).
Haematology at 1, 4, 8, 12 and 24 hours (cf. below).
FVII:C 2 to 4 samples, one in each of the following time intervals: 0-1 hour, 1-3 hours, 3-8 hours, and 8-12 hours (cf. below).
Frequent sampling: FVII:C at 30 mins, 1, 2, 3, 4, 6, 8, and 12 hours (cf. below).
PT at 1, 4, 8, 12, and 24 hours.(cf. below)
Frequent sampling: 30 min and 1, 2, 3, 4, 6, 8, 12, 18, and 24 hours. (cf. below)
Blood chemistry at 24 hours (cf. below).

### From 24 to 48 hours

Mortality and time of death.
Vital signs every 6 hours.
Number of transfusion product units required.
I.V. fluid volume including composition, e.g., colloid, crystalloid
Physical examination changes from baseline.
Number of times subject is taken into surgery and reason for surgery.
ECG at 48 hours
ARDS, infections, MOF
Adverse events

Blood sampling will be performed at 36 and 48 hours for the following:
Coagulation related parameters
Haematology
PT
Blood chemistry - only at 48 hours

### Follow-up visit - day 30

Mortality and date and time of death
Days of hospitalisation including number of days in Intensive Care Unit and of bed confinement.
Days on Ventilator
Serious Adverse Events
ARDS, infection, MOF

### ANALYSES

### Coagulation-Related Parameters and Haematology

Blood was drawn at the following time points: immediately prior to first treatment and at 1, 4, 8, 12, 24, 36, 48 hours after first treatment for the analysis of:
Coagulation-Related Parameters
APTT, Fibrinogen, D-dimers, Anti thrombin-III, F1+2, TAT
Haematology
Platelets, Haematocrit, Haemoglobin and White Blood Cells
Blood Chemistry

Blood was drawn at the following time points: prior to the first treatment and at 24, 48 hours after the first treatment for the analysis of:
S-Bilirubin, S- albumin, S-creatinine, S-potassium, S-sodium, S-alanine aminotransferase.
FVII:C (Pharmacokinetics)

Fifty subjects was frequently sampled for FVII:C, and blood was be drawn at the following time points: immediately prior to first treatment and at 30 minutes, 1, 2, 3, 4, 6, 8, and 12 hours for the analysis of FVII:C.

All other subjects had blood drawn 2-4 times, one sample in 2 to 4 of the following time intervals: 0-1 hour (immediately after first dose and before next dose is given), 1-3 hours (immediately after second dose and before next dose is given), 3-8 hours (immediately after third dose), and 8-12 hours. The samples can be taken any time in the time interval. Exact time of sampling was recorded.

### Prothrombin time

For the 50 subjects having frequent FVII:C sampling, blood was be drawn at the following time points: immediately prior to first treatment and at 30 minutes, 1, 2, 3, 4, 6, 8, 12, 18, 24, 36 and 48 hours for the analysis of Prothrombin time (PT).

All other subjects had blood drawn at the following time points: immediately prior to first treatment and at 1, 4, 8, 12, 24, 36, and 48 hours.

### Vital Signs

Vital signs was recorded prior to treatment and at 30 min, 1, 2, 4, 6, 8, 12, 16, 18 and then every 6 hours until 48 hours from first dose upon administration of the first dose of the study drug (otherwise as the condition of the subject demanded).
The following was recorded:
Body temperature (C [rectal, oral or ear])
Blood pressure (mm Hg) (systolic / diastolic) will in addition be recorded at scene of accident during pre-hospital phase.
Pulse (beats/min) will in addition be recorded at scene of accident during pre-hospital phase.
pH
Respiration rate (only when off ventilator) will in addition be recorded at scene of accident during pre-hospital phase.
Respiratory Pa02/Fi02, PaCO2
Positive end expiratory pressure (cm H20)
Glasgow coma score (cf. the present specification) was recorded at scene of accident during pre-hospital phase at trauma centre: before treatment, 24 and 48 hours from first dose of study drug. If the patient was on ventilator, the GCS was not be recorded.

### Example 3: In Vitro Evaluation of the Impact of Colloid Hemodilution, Acidosis, and Hypothermia on the Effect of Recombinant Factor VIIa

The following experiments were performed to assess the effect on clot formation of Factor VIIa under physiological conditions that are clinically relevant in trauma, ie., low pH (acidosis), low temperature (hypothermia), and colloid hemodilution.

### 1. Methods

*Blood Collection:* WB was obtained using a 21-gauge needle from six healthy volunteers. Samples were drawn into tubes containing citrate, mixing one part of citrate with nine parts of blood. The first tube of collected blood from each participant was discarded. After the blood samples had rested for 30 minutes at room temperature, they were manipulated to mimic one specific clinical situation, as outlined below.

To stimulate acidosis, WB (2 mL) was made acidic (pH 7) by the addition of 140 µL of N-2-hydroxyethylpiperazine-N'-2-ethanesulfonic acid (HEPES) 1 M buffer, adjusted to pH 7.

To simulate hypothermia, the temperature of the blood samples was lowered to 32°C. To simulate hemodilution, all solutions were mixed with citrate (10% v/v) to ensure adequate anticoagulation of the hemodiluted WB. WB was then diluted by 20%, 40%, and 60% (V/V) with one of three different colloid solutions: Human serum albumin 5%, MW = 68 000; Hetastarch 6% (Hespan^{®}, duPont Merck, Wilmington, Del, USA), MW = 450 000; or Hydroxyethyl starch (HES) 130/0.4 (Voluven^{®}, Fresenius Kabi, Bad Homburg, Germany), MW = 130 000.

*Thromboelastography Whole Blood Coagulation Analysis:* Coagulation was initiated by adding tissue factor 1:50 000 (Innovin^{®}, Dade Behring, Deerfield, III, USA) to WB and recalcifying with 15 mM calcium chloride (free CaCl₂ ∼ 2-3 mM). The final concentration of tissue factor in WB corresponded to 0.12 pM. Experiments were performed in the absence or presence of 25 nM rFVIIa (25 nM ≈ 90 µg/kg). The hemostatic process was recorded by use of a TEG coagulation analyzer (5000 series TEG analyzer, Haemoscope Corporation). The clot formation rate (CFR) was recorded as the TEG α-angle (Figure); a greater CFR value is indicative of a more robust clot formation.

*Statistical Analysis:* Pharmacodynamic parameters were summarized as means and standard deviations (SD). The student's t-test was performed on averaged data, with two-sided α set at 0.05.

### 2. Results

*Acidosis*: Lowering the pH to 7.0 significantly decreased the CFR. Addition of rFVIIa resulted in a significant increase in the CFR.

*Hypothermia*: Lowering the WB temperature to 32°C resulted in a trend toward a modest decrease of the CFR. Addition of 25 nM rFVIIa significantly increased the CFR.

### Hemodilution :

- Albumin: Hemodilution with albumin was not associated with impairment of clot formation (Table). For the 20% and 40% dilutions, but not the 60% dilution, addition of rFVIIa significantly increased the CFR.
- Hetastarch: Hemodilution with hetastarch was associated with impaired clot formation at 40% and 60% only (Table). For the 20%, but not the 40% and 60% dilutions, addition of rFVIIa significantly increased the CFR. At 60% dilution, the CFR following addition of rFVIIa remained significantly reduced compared to the CFR in normal WB.
- HES: All dilutions of WB with HES were associated with reduced CFRs relative to normal WB (Table). The addition of rFVIIa improved the CFR only at the 20% dilution level. At 40% and 60% dilution, the CFR following addition of rFVIIa remained significantly reduced compared with the CFR in normal WB. Of note, increasing the rFVIIa concentration to 200 nM (corresponding to the plasma concentration following administration of 720 µg/kg) significantly improved the CFR at 40% dilution (48±3) but failed to improve the CFR at 60% dilution, which remained significantly reduced compared with the CFR in normal WB.

### 3. Conclusions

- The in vitro clot-promoting effects of rFVIIa were not adversely affected by acidosis, hypothermia, or hemodilution below 40%. However, more severe degrees of colloid hemodilution with 6% hetastarch and HES130/0.4 impaired the effect of rFVIIa on clot formation, as measured in vitro by TEG.

| | CFR | |
|---|---|---|
| | No addition | rFVIIa |
| | | (25 nM) |
| Normal WB | 52±8 | |
| Acidosis (pH 7.0) | 44±7 | 58±6 |
| Hypothermia (32°C) | 51±9 | 59±9 |
| Hemodilution albumin 20% | 54±8 | 65±4 |
| Hemodilution albumin 40% | 55±8 | 63±4 |
| Hemodilution albumin 60% | 49±7 | 49±7 |
| Hemodilution hetastarch 20% | 51±±5 | 58±6 |
| Hemodilution hetastarch 40% | 46±4 | 49±8 |
| Hemodilution hetastarch 60% | 35±5 | 36±19 |
| Hemodilution HES 130/0.4 20% | 35±4 | 49±6 |
| Hemodilution HES 130/0.4 40% | 39±6 | 38±7 |
| Hemodilution HES 130/0.4 60% | 32±6 | 31±4 |

### Example 4: Efficacy of Factor VIIa given in conjunction with standard therapy in the treatment of trauma

The following study was performed in order to assess efficacy and safety of recombinant activated coagulation factor VII (rFVIIa, NovoSeven^{®}) as adjunctive therapy for bleeding control in severe trauma

### 1. Methods

Severely bleeding patients with severe blunt and/or penetrating trauma injury were randomized to rFVIIa (200 + 100 + 100 µg/kg) or placebo in addition to standard treatment. The first dose followed the 8th RBC (red blood cell) unit, with additional doses 1 and 3 hours later.

Patients were monitored closely during the 48-hour period following the first dose of trial product. This included recording transfusion and infusion requirements, adverse events and surgical procedures. Blood was drawn at frequent intervals to evaluate changes in coagulation and blood biochemistry parameters. Mortality, time on ventilator, hospitalization data, and serious adverse events including pre-defined critical complications (MOF and acute respiratory distress syndrome (ARDS)) as reported by the trial sites were recorded until day 30.

### Endpoints

To assess the haemostatic effect, the primary endpoint was the number of RBC units (allogeneic RBC, autologous RBC and whole blood) transfused during the 48-hour period following first dose of trial product. Outcome of therapy was further assessed through requirement for other transfusion products, mortality, days on the ventilator, and hospitalization data. Safety outcomes comprised frequency and timing of adverse events, and changes in coagulation-related laboratory variables (activated partial thromboplastin time (aPTT), platelets, fibrinogen, D-dimer, antithrombin III, prothrombin fragments 1+2, and thrombin-antithrombin complex). Because mortality is not a sensitive variable in a trauma population, we studied a composite endpoint that comprised death, MOF, and ARDS. Safety reporting on MOF and ARDS was based on pre-specified definitions provided in the study protocol.

### Statistical analysis

We calculated sample size according to the transfusion data of a retrospective study in a severe blunt trauma patient population. 14 In patients with an initial GCS ≥ 8, a 0-48h RBC transfusion requirement of 12.4 (SD: 6.2) units was found. We estimated that 70 patients in each treatment group would be required to detect a 60% reduction in 0-48h RBC requirement from 4.4 units to 1.8 units in addition to the 8 pre-dose units, with 80% power and a 5% Type 1 error. As the trial involved two trauma populations and two treatment groups, a total sample size of 280 patients was planned for. Blunt and penetrating trauma populations were analyzed separately. Results pertain to all consented and randomized patients who received trial drug. The type 1 error was set to 5%. All analyses were defined a priori, unless otherwise stated.

The total number of RBC units transfused within 48 hours from start of trial product treatment (the primary endpoint) was compared between treatment groups by use of one-sided Wilcoxon-Mann-Whitney rank test. A one-sided test was selected as it was not expected that administration of rFVIIa would increase transfusion requirements. Separate analyses were performed where patients who died within 48 hours were either excluded or assigned to the worst outcome. Priority was given to the analysis where patients who died within 48 hours were excluded because 1) in a large proportion of these patients, care was futile 2) 48-hour transfusion requirements could not be objectively assessed for patients who were alive for only a few hours. The Hodges-Lehman estimate was used to estimate the difference in RBC transfusions. Total RBC were calculated as the sum of autologous RBC, allogeneic RBC and whole blood, with each component normalized to standard units of RBC (equal to a volume of 295 mL with a haematocrit of 63%, as this was the average across all sites).

The Fisher's exact test was used for comparing the number of patients requiring massive transfusion (defined post hoc as > 20 units of RBC inclusive of the 8 pre-dose units) and number of patients experiencing MOF, ARDS, and/or death within 30 days. The relative risk reduction of massive reduction and its 95% confidence interval (CI) were calculated. Effects on 48-hour mortality were analyzed using chi-square testing.

### 2. Results

Among 301 patients randomized, 143 blunt trauma patients and 134 penetrating trauma patients were eligible for analysis. Treatment groups were well matched in terms of baseline characteristics within each of the trauma populations (Table 1). Patients were predominantly young males and were characterized by being coagulopathic, acidotic and hypothermic. Causes of penetrating trauma were primarily gunshots (68%) and stab wounds (30%), whereas 75% of blunt trauma was due to traffic-related injury.

### Bleeding control

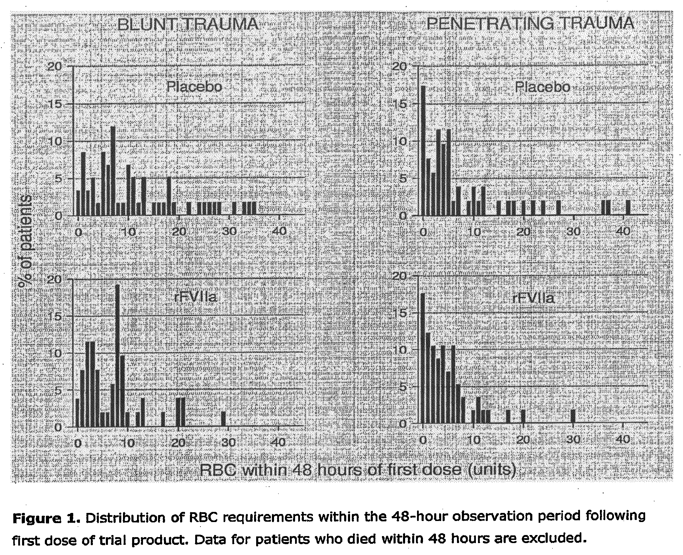

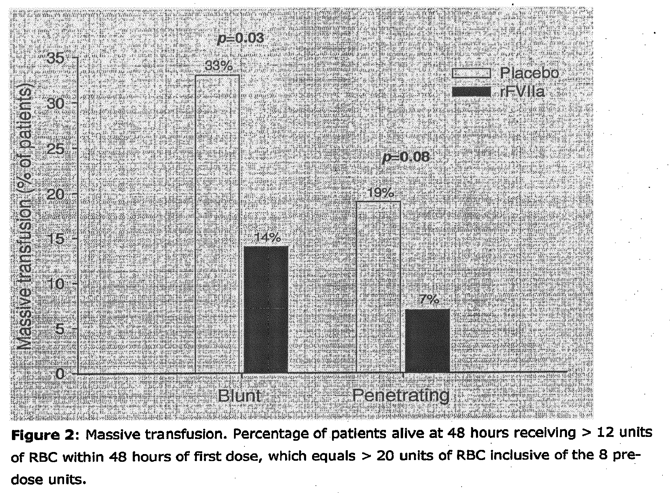

The primary endpoint, RBC requirements during the 48-hour observation period following the initial dose of trial product, is shown for patients alive at 48 hours in Figure 1. In patients with blunt trauma, rFVIIa significantly reduced 48-hour RBC requirements by 2.6 units compared with placebo (p=0.02). The need for massive transfusion was reduced from 20/61 (33%) patients in the placebo group to 8/56 (14%) in the rFVIIa group, which represents a relative risk reduction of 56% (95% CI: [9%; 79%]; p=0.03) (Figure 2). In patients with penetrating trauma, no significant effect of rFVIIa was observed with respect to 48-hour RBC requirements with an RBC reduction of 1.0 unit (p=0.10). The need for massive transfusion in penetrating trauma was reduced from 10/54 (19%) patients in the placebo group to 4/58 (7%) in the rFVIIa group, which represents a relative risk reduction of 63% (95% CI: [-12%; 88%]; p= 0.08) (**Figure 2**). When assigning worst outcome to deceased patients, statistical significance was not reached in either trauma population (**Table 2**).

No significant differences between treatment groups were observed in either trauma population with respect to administration of fresh frozen plasma, platelets, cryoprecipitate, crystalloids or colloids (data not shown).

### Clinical outcome and safety

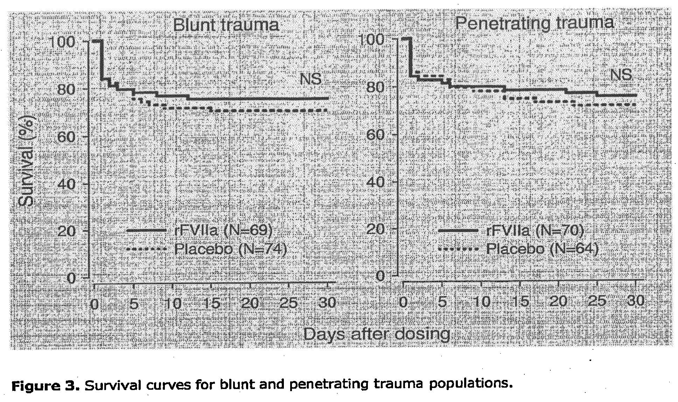

Results for adverse events, mortality, ventilator-free days and ICU-free days are summarized in **Table 3.** Positive trends in favour of rFVIIa were observed for these endpoints, especially those concerning critical complications (ARDS, MOF and/or death). Survival curves are depicted **in** **Figure 3****.**

Adverse events occurred at similar frequency and severity between treatment groups. Overall, the adverse event profile was similar between rFVIIa-treated and placebo-treated patients, and there were no apparent treatment-dependent patterns in the types of adverse events reported. As can be expected in this severely injured patient population, the three most frequently reported serious adverse events were ARDS, MOF, and sepsis.

A total of 12 thromboembolic adverse events were reported by the investigators during the trial period; 6 in rFVIIa-dosed patients and 6 in placebo-dosed patients. In patients with blunt trauma, two incidences of pulmonary embolism and a subclavian vein thrombosis (after central line) were recorded in the placebo group, whereas one jugular vein thrombosis (after central line) and one arterial limb thrombosis were recorded in rFVIIa-treated patients. In patients with penetrating trauma, one cerebral infarction and one DVT was noted in each treatment group. In addition, a mesenteric vein thrombosis was recorded in the placebo group and an intestinal infarction (at the site of operation) and an event of phlebothrombosis was noted in the rFVIIa group. All 12 thromboembolic events were reported as serious adverse events.

### 3. Conclusions

rFVIIa assisted in the control of bleeding in severe blunt trauma and resulted in a significant reduction in RBC transfusion. Similar trends were observed in penetrating trauma. The safety of rFVIIa was established in this trauma population within the investigated dose range

**Table 1: Baseline Characteristics**

| | **Blunt trauma** | | **Penetrating trauma** | |
|---|---|---|---|---|
| Variable | Placebo (N=74) | rFVIIa (N=69) | Placebo (N = 64) | rFVIIa (N=70) |
| Male sex | 52 (70%) | 48 (70%) | 60 (94%) | 66 (94%) |
| Aqe (years) | 35 ± 13 | 33 ± 13 | 32 ± 10 | 29 ± 10 |
| ISS | 32 ± 13 | 34 ± 12 | 26 ± 11 | 26 ± 15 |
| Number of ISS body regions injured* | | | | |
| 1 | 4 (5%) | 6 (9%) | 25 (39%) | 21 (30%) |
| 2-3 | 36 (49%) | 29 (42%) | 36 (56%) | 43 (61%) |
| > 3 | 32 (43%) | 33 (48%) | 3 (5%) | 6 (9%) |
| Glasgow Coma Score | | | | |
| ≤ 8 | 8 (11%) | 8 (12%) | 5 (8%) | 3 (4%) |
| 9-12 | 18 (24%) | 11 (16%) | 8 (13%) | 6 (9%) |
| 13-15 | 48 (65%) | 47 (68%) | 51 (80%) | 60 (86%) |
| Time from injury to hospitalization | | | | |
| 0-1 hours | 27 (36%) | 21 (30%) | 40 (63%) | 41 (59%) |
| 1-2 hours | 23 (31%) | 23 (33%) | 10 (16%) | 8 (11%) |
| 2-4 hours | 10 (14%) | 11 (16%) | 3 (5%) | 3 (4%) |
| > 4 hours | 7 (9%) | 3 (4%) | 1 (2%) | 5 (7%) |
| Unknown | 7 (9%) | 11 (16%) | 10 (16%) | 13 (19%) |
| Time from hospitalization to trial product dosing | | | | |
| 0-2 hours | 15 (20%) | 19 (28%) | 8 (13%) | 11 (16%) |
| 2-4 hours | 24 (32%) | 18 (26%) | 23 (36%) | 25 (36%) |
| 4-6 hours | 17 (23%) | 12 (17%) | 16 (25%) | 15 (21%) |
| > 6 hours | 17 (23%) | 18 (26%) | 17 (27%) | 19 (27%) |
| Unknown | 1 (1%) | 2 (3%) | 0 (0%) | 0 (0%) |
| Vital signs | | | | |
| Systolic arterial blood pressure (mmHg) | 111 ± 27 | 102 ± 24 | 114 ± 25 | 111 ± 24 |
| Body temperature (°C) | 35.3 ± 1.6 | 35.2 ± 1.6 | 35.2 ± 1.2 | 35.3 ± 1.3 |
| Biological variables | | | | |
| Hemoglobin (g/dL) | 9.1 ± 2.8 | 9.3 ± 2.5 | 8.8 ± 3.0 | 8.5 ± 2.8 |
| pH | 7.26 ± 0.11 | 7.24 ± 0.13 | 7.28 ± 0.11 | 7.27 ± 0.09 |
| aPTT (seconds) | 51 ± 28 | 49 ± 24 | 54 ± 26 | 49 ± 27 |
| PT (seconds) | 19 ± 6 | 20 ± 8 | 22 ± 6 | 18 ± 5 |

| | | | | |
|---|---|---|---|---|
| Data intervals refer to means ± SD. Other data refer to number (and percentage) of patients. * Body regions as defined for the Injury Severity Scale. | | | | |

No significant differences between rFVIIa and placebo groups were observed. aPTT: activated partial thromboplastin time; PT: prothrombin time

**Table 2: Total RBC transfusions (units) during 48 hours after first dose of trial drug**

| | **Placebo** | | **rFVIIa** | | | |
|---|---|---|---|---|---|---|
| | n | Median (range) | n | Median (range) | Estimated RBC reduction * | *p* |
| ***Blunt*** | N=74 | | N=69 | | | |
| All patients | 72 | 7.2 (0-35) | 64 | 7.8 (0-48) | 2.00^{†} | 0.07 ^{†} |
| Alive at 48h | 59 | 7.5 (0-35) | 52 | 7.0 (0-29) | 2.60 | 0.02 |
| ***Penetrating*** | N=64 | | N=70 | | | |
| All patients | 61 | 4.8 (0-41) | 69 | 4.0 (0-37) | 0.20^{†} | 0.24 ^{†} |
| Alive at 48h | 52 | 4.2 (0-41) | 57 | 3.9 (0-30) | 1.00 | 0.10 |

| | | | | | | |
|---|---|---|---|---|---|---|
| *Hodges-Lehman point estimate of the shift in transfusion amount from placebo to active group. ^{†}Patients who died within 48 hours were assigned the highest rank. | | | | | | |

**Table 3: Adverse events and clinical outcomes**

| | **Blunt trauma** | | | **Penetrating trauma** | | |
|---|---|---|---|---|---|---|
| | Placebo (N=74) | rFVIIa (N=69) | | Placebo (N=64) | rFVIIa (N=70) | |
| Serious adverse events | | | | | | |
| Patients with events | 49 (66%) | 44 (64%) | | 36 (56%) | 36 (51%) | |
| Number of events | 109 | 91 | | 76 | 59 | |
| Thromboembolic adverse events | | | | | | |
| Patients with events | 3 (4%) | 2 (3%) | | 3 (5%) | 4 (6%) | |
| Number of events | 3 | 2 | | 3 | 4 | |
| | | | | | | |
| 48-hour mortality | 13 (18%) | 13 (19%) | *p* = 0.84 | 10 (16%) | 12 (17%) | *p* = 0.85 |
| Patients with critical complications within 30 days^{†} | | | | | | |
| ARDS | 12 (16%) | 3 (4%) | *p*=0.03 | 5 (8%) | 4 (6%) | *p*=0.74 |
| MOF | 9 (12%) | 5 (7%) | *p*=0.41 | 7 (11%) | 2 (3%) | *p*=0.09 |
| Death | 22 (30%) | 17 (25%) | *p*=0.58 | 18 (29%) | 17 (25%) | *p*=0.69 |
| Patients with ARDS, MOF, and/or death | 31 (42%) | 20 (29%) | *p*=0.16 | 22 (34%) | 20 (29%) | *p*=0.57 |
| | | | | | | |
| Ventilator-free days (median and range) | 14 (0-30) | 17(0-30) | *p*=0.53 | 21 (0-30) | 26 (0-30) | *p*=0.18 |
| ICU-free days (median and range) | 8 (0-29) | 13 (0-30) | *p*=0.22 | 19 (0-30) | 23 (0-30) | *p*=0.28 |

| | | | | | | |
|---|---|---|---|---|---|---|
| MOF: Multiple organ failure; ARDS: Acute respiratory distress syndrome; ICU: Intensive care unit | | | | | | |

## Claims

1. Use of Factor VIIa, or a Factor VIIa polypeptide that exhibits at least 70% of the specific biological activity of FVIIa, for the manufacture of a medicament for preventing or attenuating multiple organ failure (MOF) or acute respiratory distress syndrome (ARDS) in trauma patients that have received transfusions of less than 10 units of packed red blood cells (PRBC), and wherein said transfusions are made between the time of patient's traumatic injury and the time of administration of Factor VIIa or Factor VIIa equivalent.

2. Use according to claim 1, wherein said FVIIa polypeptide is V158D/E296V/M298Q-FVII(a).

3. Use according to any one of claims 1 to 2, wherein the patient is suffering from blunt trauma.

4. Use according to any one of claims 1 to 2, wherein the patient is suffering from penetrating trauma.

5. Use according to any one of claims 1 to 4, wherein the medicament comprises at least 150 µg/kg of Factor VIIa, or a corresponding amount of a Factor VIIa polypeptide that exhibits at least 70% of the specific biological activity of FVIIa.

6. Use according to claim 5, wherein the medicament is for administration in a first dose containing at least 150 µg/kg, preferably 200 µg/kg Factor VIIa, or a corresponding amount of a Factor VIIa polypeptide that exhibits at least 70% of the specific biological activity of FVIIa, followed by a second dose containing 100 µg/kg Factor VIIa, or a corresponding amount of a Factor VIIa polypeptide that exhibits at least 70% of the specific biological activity of FVIIa, administered one hour after the start of treatment.

7. Use according to claim 6, wherein a further, third dose containing 100 µg/kg Factor VIIa, or a corresponding amount of a Factor VIIa polypeptide that exhibits at least 70% of the specific biological activity of FVIIa, is administered three hours after the start of treatment.

## Patentansprüche

1. Verwendung von Faktor VIIa oder eines Faktor-VIIa-Polypeptids, das mindestens 70% der spezifischen biologischen Aktivität von FVIIa zeigt, zur Herstellung eines Medikaments zur Prävention oder Abschwächung von Multiorganversagen (Multiple Organ Failure, MOF) oder Atemnotsyndrom des Erwachsenen (Acute Respiratory Distress Syndrome, ARDS) bei Traumapatienten, die Transfusionen von weniger als 10 Einheiten Erythrozytenkonzentrat (Packed Red Blood Cells, PRBC) erhielten, und wobei die Transfusionen zwischen dem Zeitpunkt der traumatischen Verletzung des Patienten und dem Zeitpunkt der Verabreichung von Faktor VIIa oder Faktor-VIIa-Äquivalent erfolgen.

2. Verwendung nach Anspruch 1, wobei es sich bei dem FVIIa-Polypeptid um V158D-E296V-M298Q-FVII(a) handelt.

3. Verwendung nach Anspruch 1 oder 2, wobei der Patient an einem stumpfen Trauma leidet.

4. Verwendung nach Anspruch 1 oder 2, wobei der Patient an einem penetrierenden Trauma leidet.

5. Verwendung nach einem der Ansprüche 1 bis 4, wobei das Medikament mindestens 150 µg/kg Faktor VIIa oder eine entsprechende Menge eines Faktor-VIIa-Polypeptids, das mindestens 70% der spezifischen biologischen Aktivität von FVIIa zeigt, umfasst.

6. Verwendung nach Anspruch 5, wobei das Medikament für die Verabreichung in einer ersten Dosis, die mindestens 150 µg/kg, vorzugsweise 200 µg/kg Faktor VIIa oder eine entsprechende Menge eines Faktor-VIIa-Polypeptids, das mindestens 70% der spezifischen biologischen Aktivität von FVIIa zeigt, enthält, gefolgt von einer zweiten Dosis, die 100 µg/kg Faktor VIIa oder eine entsprechende Menge eines Faktor-VIIa-Polypeptids, das mindestens 70% der spezifischen biologischen Aktivität von FVIIa zeigt, enthält, und die eine Stunde nach Beginn der Behandlung verabreicht wird, bestimmt ist.

7. Verwendung nach Anspruch 6, wobei eine weitere dritte Dosis, die 100 µg/kg Faktor VIIa oder eine entsprechende Menge eines Faktor-VIIa-Polypeptids, das mindestens 70% der spezifischen biologischen Aktivität von FVIIa zeigt, enthält, drei Stunden nach Beginn der Behandlung verabreicht wird.

## Revendications

1. Utilisation du facteur VIIa, ou d'un polypeptide de facteur VIIa qui présente au moins 70 % de l'activité biologique spécifique de FVIIa, pour la fabrication d'un médicament pour prévenir ou atténuer une insuffisance d'organes multiples (MOF) ou le syndrome de détresse respiratoire aigu (SDRA) chez des patients traumatisés qui ont reçu des transfusions de moins de 10 unités de globules rouges concentrés (PRBC), et où lesdites transfusions sont effectuées entre le moment de la lésion traumatique du patient et le moment d'administration de facteur VIIa ou d'équivalent de facteur VIIa.

2. Utilisation selon la revendication 1, dans laquelle ledit FVIIa polypeptide est V158D/E296V/M298Q-FVII(a).

3. Utilisation selon l'une quelconque des revendications 1 à 2, dans laquelle le patient souffre d'un traumatisme contondant.

4. Utilisation selon l'une quelconque des revendications 1 à 2, dans laquelle le patient souffre d'un traumatisme pénétrant.

5. Utilisation selon l'une quelconque des revendications 1 à 4, dans laquelle le médicament comprend au moins 150 µg/kg de facteur VIIa, ou une quantité correspondante d'un polypeptide de facteur VIIa qui présente au moins 70% de l'activité biologique spécifique de FVIIa.

6. Utilisation selon la revendication 5, dans laquelle le médicament est pour administration dans une première dose contenant au moins 150 µg/kg, de préférence 200 µg/kg de facteur VIIa, ou une quantité correspondante d'un polypeptide de facteur VIIa qui présente au moins 70 % de l'activité biologique spécifique de FVIIa, suivi par une deuxième dose contenant 100 µg/kg de facteur VIIa, ou une quantité correspondante d'un polypeptide de facteur VIIa qui présente au moins 70 % de l'activité biologique spécifique de FVIIa, administré une heure après le début du traitement.

7. Utilisation selon la revendication 6, dans laquelle une troisième dose supplémentaire contenant 100 µg/kg de facteur VIIa, ou une quantité correspondante d'un polypeptide de facteur VIIa qui présente au moins 70 % de l'activité biologique spécifique de FVIIa, est administré trois heures après le début du traitement.
